(19) Europäisches Patentamt
European Patent Office
Office européen des brevets

(11) Publication number: **0 114 498**
**B1**

# (12) EUROPEAN PATENT SPECIFICATION

(45) Date of publication of patent specification: **07.01.87**

(21) Application number: **83307772.0**

(22) Date of filing: **20.12.83**

(51) Int. Cl.[4]: **C 07 C 1/20,** C 07 C 11/02 // B01J29/28

## (54) Conversion of alcohols and/or ethers to olefins.

(30) Priority: **17.01.83 US 458401**

(43) Date of publication of application:
**01.08.84 Bulletin 84/31**

(45) Publication of the grant of the patent:
**07.01.87 Bulletin 87/02**

(84) Designated Contracting States:
**BE DE FR GB IT NL**

(56) References cited:
**DE-A-2 755 229**
**DE-A-2 928 922**
**US-A-3 979 472**
**US-A-4 060 568**
**US-A-4 148 835**
**US-A-4 311 865**
**US-A-4 358 397**

(73) Proprietor: **MOBIL OIL CORPORATION**
**150 East 42nd Street**
**New York New York 10017 (US)**

(72) Inventor: **Chu, Cynthia Ting-Wah**
**283 Westcott Boulevard**
**Pennington New Jersey 08534 (US)**
Inventor: **Chang, Clarence Dayton**
**11 Murray Place**
**Princeton New Jersey 08540 (US)**
Inventor: **Miale, Joseph Nicolas**
**25 Merritt Drive**
**Lawrenceville New Jersey 08648 (US)**

(74) Representative: **West, Alan Harry**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 114 498 B1**

**Description**

This invention relates to the conversion of alcohols and/or ethers to olefins in the presence of a zeolite catalyst.

The petrochemical industry has undergone tremendous growth in the past few decades. The production of synthetic fibers, plastics and petrochemicals by this and allied industries has steadily grown, at least in part, because of the availability of increasing supplies of inexpensive petrochemical raw materials, such as ethylene, propylene and other olefins. The principal raw material for ethylene at the present time is petroleum naphtha, which is steam cracked to produce a mixture of products from which ethylene is recovered. The manufacture of polyethylene and styrene monomers utilizes a significant portion of this ethylene feed.

The ever-increasing demand for olefinic feedstocks has periodically caused a shortage of these basic raw materials either because of a limitation in petroleum feedstocks of suitable quality or limitation in naphtha cracking capacity. An alternative source of ethylene from non-petroleum sources is one obvious means of keeping pace with the demand for ethylene and other olefins.

In recent years various workers have reported that methanol and/or dimethyl ether, which may be obtained from coal, natural gas or biomass, can be converted to more complex hydrocarbons, such as olefins and aromatics, by using a particular group of zeolite catalysts, exemplified by ZSM—5 zeolite. Ethylene is one of the olefinic hydrocarbons which may be obtained in this catalytic conversion. The reaction is highly exothermic and the olefins initially formed have a tendency to undergo further reaction to produce aromatic hydrocarbons useful in the production of motor gasoline. Factors which improve ethylene selectivity in the co-production of ethylene plus gasoline from methanol and/or dimethyl ether over zeolite catalyst include low diffusivity, low methanol partial pressure, dilution of the feed stream, partial methanol conversion and crystal size of the zeolite catalyst.

For example, high ethylene selectivities in the conversion of lower alcohols over zeolite catalysts are achieved in the presence of small-pore crystalline aluminosilicate zeolites such as erionite and ZSM—34. Examples of this form of catalytic conversion can be found in U.S. Patent Nos. 4,062,905 which discloses the use of erionite and 4,079,096 which discloses methanol conversion over ZSM—34. U.S. Patent No. 4,025,572 discloses that ethylene selectivity can be improved by diluting ZSM—5 with an inert diluent. A similar result is achieved, according to U.S. Patent No. 4,025,575 through the use of subatmospheric partial pressure of the feed. Selectivity of ethylene is also improved by employing ZSM—5 zeolite in large crystal form of at least about one micron. Better selectivity is also obtained by interdispersing amorphous silica within the interior of the crystalline structure of the zeolite catalyst as disclosed in U.S. Patent Nos. 4,060,568 and 4,100,219.

U.S. Patent No. 4,148,835 discloses a process for forming light olefinic hydrocarbons from the conversion of lower alcohols over a crystalline aluminosilicate having a crystal size of at least about one micron, in which the zeolite contains a Group IIB, a Group VIII metal and magnesium. The metals are exchanged within the zeolite from aqueous solutions of metal salts.

Generally, it is well known to enhance the catalytic properties of aluminosilicates by the addition of metals. Aluminosilicates have been activated, i.e., metal-loaded, for various petroleum, chemical and enzymatic reactions by methods employing impregnation, vapor deposition and base-exchange of the desired metal to be loaded. The use of aqueous solutions, especially those of polyvalent metal salts is the standard method employed to exchange the metals into the crystalline aluminosilicate structure.

However, the use of aqueous solutions severely limits the availability of those skilled in the art to exchange into the crystalline aluminosilicate structure many catalytically important metals.

Cation exchange may be thwarted or prevented, since, in the aqueous media conventionally employed, one or more of the following phenomena occur:

(1) The cation is converted to an anion in aqueous solution.

(2) Compounds containing the cation are insoluble in aqueous solutions, thus no cations are formed in solution.

(3) Compounds containing the cation in solution of aqueous media result in low pH conditions that destroy some aluminosilicates entirely, and other aluminosilicates partially.

(4) Compounds containing the cation in a complex form which are soluble in aqueous media and able to penetrate large pore zeolites, are too large to penetrate the pores of many of the other zeolites so that shape-selective catalysts with these cations are impossible or the impregnation of such metals is insufficient such that the metal is deposited on the external surface of the zeolite or in the matrix. Often in metal-containing zeolites the metals are not adequately anchored within the zeolite channels. Under the severe conditions of temperature and pressure encountered in catalysis, the occluded metals migrate from the zeolite channels to the zeolite surface.

(5) Hydrophobicity is also a limiting factor in most metal loading using aqueous solutions, especially metal loading in hydrophobic aluminosilicates having high $SiO_2/Al_2O_3$ mole ratios, e.g., $SiO_2/Al_2O_3$ greater than about 20, including aluminum-free porotectosilicates.

Both US—A3 979 472 and DE—A—2 755 229 describe the production of lower olefins from methanol or dimethylether over catalysts comprising metal-loaded zeolites. The former describes only the treatment of HZSM—5 zeolite with antimony trimethoxide in solution in para-xylene, and the latter describes the

treatment of zeolite 13X — a large pore zeolite — with various metals in various solvents, but mentions specifically only water, methanol, formamide, dimethylformamide and mixtures thereof.

It has now been found that metals can be loaded into the zeolite structure by the use of polar non-aqueous inorganic solutions such as liquid ammonia. The use of such non-aqueous inorganic solutions allows a greater metal inclusion and stability such that metal-containing zeolites prepared from the non-aqueous inorganic solutions tend to have increased activity and incur less metal migration from the zeolite pores than zeolites loaded with metals from aqueous media.

In accordance with the present invention, there is provided a process for converting a charge comprising an alcohol and/or ether to an olefinic product in which the charge is contacted under conversion conditions with a catalyst comprising a crystalline aluminosilicate zeolite having incorporated therein at least one metal or metal compound by contacting the zeolite with a solution of the metal or metal compound and recovering the resulting metal-exchanged or loaded crystalline aluminosilicate zeolite, characterized in that the metal or metal compound is in solution in a non-aqueous inorganic solvent.

Preferably the solvent is liquid ammonia.

It is contemplated that a feed comprising any monohydric alcohol having from 1 to 4 carbon atoms may be used in the present process. Thus, methanol, ethanol, n-propanol, isopropanol, n-butanol, sec-butanol and isobutanol may be used alone or in admixture with one another. The particularly preferred feed is methanol. The corresponding aliphatic ethers, e.g., dimethyl ether or mixtures thereof or in admixture with the monohydric alcohols are also useful.

Such a feed is brought into contact, under conversion conditions, with a catalyst comprising a crystalline aluminosilicate zeolite having at least one metal or metal compound which has been incorporated into the zeolite by means of a non-aqueous inorganic solvent. In general, the catalyst useful for converting the monohydric alcohol and/or ether to an olefin rich product material comprise a ZSM—5 type crystalline zeolite or a small pore crystalline zeolite. The ZSM—5 type zeolite catalyst used herein is a crystalline zeolite having a silica/alumina ratio greater than 12 and a constraint index between 1 and 12. Zeolites of this defined type and their use as conversion catalysts for lower aliphatic alcohols are described in the U.S. patents referred to above, particularly U.S. Patent Nos. 3,984,106, 4,025,571, 4,058,576 and 4,148,835.

The preferred class of zeolite starting materials described above are ZSM—5 type zeolites as exemplified by ZSM—5, ZSM—11, ZSM—12, ZSM—23, ZSM—35 and ZSM—38, with ZSM—5 being particularly preferred.

ZSM—5 is more particularly described in U.S. Patent No. 3,702,886. ZSM—11 is more particularly described in U.S. Patent No. 3,709,979. ZSM—12 is more particularly described in U.S. Patent No. 3,832,449. ZSM—23 is more particularly described in U.S. Patent No. 4,076,842. ZSM—35 is more particularly described in U.S. Patent No. 4,016,245. ZSM—38 is more particularly described in U.S. Patent No. 4,046,859.

Particularly preferred catalyst starting materials within the above descriptions are ZSM—5 type zeolite catalysts made with large crystal ZSM—5, i.e., a crystal size of at least 1 micron, as described in U.S. Patent Nos. 4,025,571 and 4,148,835.

In addition to ZSM—5 zeolites other zeolites known in the art as small pore crystalline aluminosilicate zeolites may be employed. These small pore zeolites may be either naturally occurring or synthetic and include, by way of example, erionite, chabazite, Zeolite T, Zeolite ZK—5, and Zeolite ZSM—34. Zeolite T is described in U.S. Patent No. 2,950,952, Zeolite ZK—5 in U.S. Patent No. 3,427,195, and ZSM—34 in U.S. Patent Nos. 4,079,095 and 4,079,096.

Preferred aluminosilicate zeolites useful in the invention are those zeolites as defined above and having a silica-to-alumina mole ratio of at least 20, and, more preferably, at least 70. Such high-silica zeolites can be characterized as hydrophobic and thus are limited with respect to the degree of metal loading or ion exchange using conventional aqueous systems as the metal carrier.

The crystalline aluminosilicate starting material as described above is modified by incorporating at least one metal or metal compound therein for use in converting the monohydric alcohol and/or respective dialkyl ether to an olefinic product. In particular, the crystalline aluminosilicate starting material is contacted with a non-aqueous inorganic solvent solution of the metal or metal compound in cationic form and subsequently recovering the resulting cationic-exchanged or metal-loaded crystalline aluminosilicate. The solvent solution is preferably anhydrous, although minimal water may be present with the non-aqueous inorganic solvent without adverse effect on cation-exchange. The metals which can be incorporated in those aluminosilicate zeolites which have been found useful in the conversion of monohydric alcohols to olefins include metals selected from Group I—VIII metals as set forth in the Periodic Table of elements. Preferred metals include alkali and alkaline earth of Groups I and IA, respectively, including Na, K, Ca, Mg and Ba.

Transition metals such as Ga and Ce as well as Group VIII metals including palladium are further examples of metals which can be incorporated into the zeolites useful in the present conversion process.

In addition, more than one metal or metal compound can be incorporated into the useful zeolites. For example, multimetallites such as those disclosed in U.S. Patent No. 3,752,776 have been a suggested means of incorporating multiple metal compounds within zeolites. Multimetallites are a class of inorganic materials having the ability to serve as catalysts in a wide range of hydrocarbon conversion reactions. The

empirical formula of these compounds can be written:

$$Mx_1\ Mx_2\ Mx_3 \ldots Mn_n\ Oy$$

where $Mx_1$, $Mx_2$, $Mx_3$ and $Mx_n$ are selected from the group consisting of catalytically active metals. The subscripts of the various symbols should reflect the empirical composition using the Law of Definite Proportions, i.e., the smallest whole numbers.

U.S. Patent No. 3,752,776 discloses a method for preparing multimetallite catalyst by thermally decomposing inorganic compounds known as heteropoly amines which comprise a transition metal complex and a heteropoly anion. These compounds can be represented by the general empirical formula:

$$[ML_N]_{m/n}\ [M'_xM''_yO_z]$$

where M = a transition metal or a mixture of a transition metal with hydrogen
N = the number of ligands
$L_N$ = a set of N ligands at least one of which functions as a reducing agent
m = the charge on the anion
n = the charge on the cation
M' = a transition metal or non-metal or hydrogen
M" = a metal selected from the group consisting of Group VB and VIB metals
x is 1 or 2
y is between 5 and 20
z is between 20 and 50

Thermal decomposition of these compounds produce multimetallites and materials which can be represented by the following formula:

$$M_{m/n}\ M'_x\ M''_y\ O_w$$

where M is derived from the cation of a compound of the above type wherein said cation is hydrogen, or a transition metal or mixture thereof with each other, M' is a metal or a non-metal and M" is derived from the metal which is represented by M" in the parent compound and w is a number representing the number of oxygen atoms and is less than z noted hereinbefore. Generally at least two of the M, M' and M" differ from each other. For purposes of illustration the products of the thermal decomposition of the compounds:

$$[Co(NH_3)_6]\ [MMo_6O_{24}H_6] \times H_2O$$

wherein M = Co, Fe, Cr and Al should be written as follows:

$CoCOMo_6O_{19}$
$CoFeMo_6O_{19}$
$CoCrMo_6O_{19}$
$CoAlMo_6O_{19}$

In addition to the above-described heteropoly amine precursors, multimetallites can also be derived from a mixture of metal salts and/or metal oxides, the mixture containing at least two different metal components and exhibiting catalytic activity when incorporated within a zeolite.

The preferred non-aqueous inorganic solvent employed to disperse the metal or metal compounds within the zeolite pore structure is liquid ammonia with anhydrous liquid ammonia, particularly preferred. Other inorganic non-aqueous solvents which are useful include polar normally gaseous compounds such as sulfur dioxide, and fused inorganic salts.

The metal or metal compounds are dispersed in the non-aqueous solvents as cations of a salt which is soluble in the non-aqueous solvent utilized. Representative of the anions of the catalytically active metal salts which can be employed to base exchange or metal-load the aluminosilicate zeolites include chlorides, bromides, iodides, carbonates, biocarbonates, sulfates, sulfides, thiocyanates, dithiocarbonates, peroxysulfates, acetates, benzoates, oxalates, citrates, florides, nitrates, nitrites and formates. The preferred salts are chlorides, nitrates, sulfates and acetates. Nitrates are particularly preferred when liquid ammonia is used as the solvent dispersing medium.

The range of concentration of cation to solvent is not particularly critical, zince the lower the concentration of cation, the more contacts of the zeolite with the solution are required, and conversely, the higher the concentration less contact time is required. Typically the concentration may vary between 0.1 weight percent to saturation. The amount of catalytically active metal component incorporated within the crystalline structure of the aluminosilicate will vary depending upon the particular metal or metals incorporated. Generally, the amount of each metal component will be within the range of 0.05 to 40% by weight. It will be understood that the amount of metal component or components will be such as to afford

the conversion of monohydric alcohol and/or ether charge to a product which contains a major proportion of olefins.

The metal salts, such as metal nitrate, are introduced within the zeolite by dissolving the metal salt in the non-aqueous solvent such as liquid ammonia and contacting the zeolite with the formed solution. The non-aqueous solvent may contain some water although it is preferred that the solvent utilized contain greater than 50 weight percent of non-aqueous component. The solution permeates the zeolite structure resulting in intrazeolite occlusion of the metal salts. The zeolite is then heated under generally mild temperatures ranging from room temperature to 400°F (240°C), preferably 230°F to 300°F (110°C to 149°C), in order to drive off the solvent. The resulting dry composition is then calcined at temperatures ranging from 75°F to 1200°F (24°C to 649°C) for periods ranging from 0.5 to 24 hours, preferably 1 to 4 hours. Such calcining converts the metal salts to free metal resulting in zeolite compositions containing virtually immobilized occluded metals.

Multimetallite precursors suitable for incorporation into the zeolite compositions of the present invention include the metal amine heteropoly salts described in U.S. Patent No. 3,752,776. Multimetallite precursors may also include a mixture of metal salts and/or metal oxides, the mixture containing at least two metal species, such as precursors being described in greater detail above.

Metal salts suitable for inclusion and multimetallite precursors include those described above such as metal chlorides and metal nitrates as well as ammonium metal oxides. Preferred metal salts include hydrated metal nitrates such as $Zn(No_3)\cdot 6H_2O$ and $Ce(No_3)_3\cdot 9H_2O$. Metal oxides or metal chlorides such as $MoO_3$ and $GaCl_3$ may supplement or replace metal salts in a multimetallite precursor.

A multimetallite precursor is introduced within the zeolite in the same manner described above by dissolving the precursors in a non-aqueous solvent and contacting the zeolite with the solvent-multimetallite solution. The zeolite is then heated to drive off the solvent and calcined to convert the multimetallite precursors to multimetallites.

In one embodiment, the zeolite is combined with all the components of a multimetallite precursor, e.g., a mixture of hydrated metal nitrates. The non-aqueous solvent is then added to the zeolite-multimetallites precursor mixture. Addition of the solvent dissolves the multimetallite precursor and the resulting solution permeates the zeolite structure resulting in intrazeolite occlusion of the multimetallite precursors whereupon the solvent is driven off by heating and the precursors converted to multimetallites by calcination.

In another embodiment each metal species of a multimetallite is independently incorporated and calcined within the zeolite composition as described above. For example, a single metal hydrate is introduced within the zeolite by the non-aqueous solvent. The resulting composite is dried and calcined and another metal salt is subsequently introduced in a similar fashion. The precursor occluded zeolite is then calcined to form a multimetallite occluded zeolite.

The catalyst composition may be in the form of compacted pellets or extrudate particles with a diameter, for example, of 1/8 inch (0.3 cm) or 1/4 inch (0.6 cm); or in the form of beads; or in the form of fine particles of 50 microns diameter suitable for fluidization. The particular form chosen is determined by the type of catalyst bed to be used, which may be fixed, compacted, a fixed fluidized bed, or a variety of transport bed. In any case, the crystalline aluminosilicate zeolite component of the catalyst composition may be blended with a binder such as alumina or silica-alumina to form the above-described particles by methods well known to those skilled in the art of catalyst preparation.

In the conversion process, the feed consisting essentially of one or more of the alcohols and/or ethers described previously is contacted with the above-described metal-containing catalyst at a temperature of 500°F to 1000°F (260°C to 538°C) and preferably 700°F to 950°F (371°C to 510°C), a liquid hourly space velocity (LHSV) of 1 to 100 and at an absolute pressure of 0.1 to 30 atmospheres (10 to 3000 kPa), preferably between 0.5 and 10 atmospheres (50 to 1000 kPa).

The reaction product effluent from the process contains steam and a hydrocarbon mixture particularly rich in the light olefins, ethylene, propylene and butene with some aromatic hydrocarbons. Generally, a major fraction of the total olefins, calculated on a mole basis, is ethylene plus propylene. The predominant aromatic hydrocarbons are monocyclic hydrocarbons, notably $C_8$ and $C_9^+$ aromatics with a high proportion of paraxylene. The olefinic product is useful as a raw material for the manufacture of important chemicals used in the production of synthetic fibers and plastics. Additionally, the olefin product can be subsequently converted to olefinic gasoline and higher boiling distillate boiling range hydrocarbons such as fuel oil by passage of the olefin product over ZSM—5 type catalysts at the appropriate conversion conditions. This process is known and described in U.S. Patent Nos. 4,254,295 and 4,150,062.

The following examples illustrate the process of this invention. In the examples, reference is made to the alpha activity of the metal-containing zeolite. Alpha activity is a measure of normal hexane cracking conversion with respect to a high activity silica-alumina cracking catalyst. The method of determining alpha is more fully described in the *Journal of Catalysis,* Vol. VI, pp. 278—287, 1966.

Example 1

A 3 g sample of low-sodium HZSM—5 ($SiO_2/Al_2O_3 \sim 65$) was mixed with 0.56 g $Ca(NO_3)_2\cdot 4H_2O$ (enough for 1:1 Ca:framework Al atomic ratio). Anhydrous liquid ammonia was added with continuous stirring until the solids were completely immersed. The ammonia was allowed to evaporate with final

degassing for 30 minutes at 130°C. It was then calcined at 538°C and tested for hexane cracking (α = 34). The resultant CaZSM—5 was then tested for methanol conversion activity and the results are shown in Table 1.

Example 2

Preparation as in Example 1 except that 6 g HZSM—5 and 0.8 g $Mg(NO_3)_2 \cdot 6H_2O$ (enough for 1:1 Mg:Al) were used (α = 50). Again the methanol conversion data for the resultant catalyst are given in Table 1.

Example 3

Preparation as in Example 1 except that 5 g HZSM—5 and 0.6 g $Ba(NO_3)_2$ were used (Ba/Al = 1) (α = 83). Again Table 1 gives the results of using the resulting catalyst to effect conversion of methanol.

TABLE 1

| Example No. | 1 | 2 | 3 |
|---|---|---|---|
| Temperature °C | 500 | 500 | 500 |
| Pressure, atms. | 14.70 | 14.70 | 14.70 |
| (kPa) | 1485 | 1485 | 1485 |
| LHSV | 1.00 | 1.00 | 1.00 |
| Time on Stream (TOS) | 2.50 | 6.50 | 30.75 |
| | Product Distribution (wt.%) | | |
| $H_2$ | 51.10 | 56.02 | 55.30 |
| DME | 0.45 | 0.24 | 0.19 |
| MeOH | 3.47 | 0.47 | 0.01 |
| CO | 2.43 | 0.63 | 1.01 |
| $CO_2$ | 0.54 | 0.58 | 0.43 |
| $H_2$ | 0.51 | 0.38 | 0.19 |
| HC | 41.48 | 41.67 | 42.88 |
| | Hydrocarbon Distribution (wt.%) | | |
| Methane | 1.97 | 3.30 | 5.45 |
| Ethane | 0.38 | 2.46 | 1.04 |
| Ethylene | 15.61 | 15.01 | 14.01 |
| Propane | 3.90 | 16.04 | 7.77 |
| Propylene | 39.42 | 20.98 | 20.77 |
| i-butane | 3.67 | 8.59 | 4.87 |
| n-butane | 1.11 | 3.65 | 2.37 |
| Butenes | 18.42 | 9.51 | 11.43 |
| i-pentane | 1.86 | 2.84 | 2.18 |
| n-pentane | 0.38 | 0.33 | 0.60 |

TABLE 1 Continued

| Example No. | 1 | 2 | 3 |
|---|---|---|---|
| | Hydrocarbon Distribution (wt.%) | | |
| Pentenes | 3.28 | 1.91 | 3.31 |
| $C_6$+ PON | 2.62 | 2.95 | 3.55 |
| Benzene | 0.08 | 0.58 | 1.25 |
| Toluene | 1.08 | 3.28 | 6.09 |
| $C_8$ Aromatics | 3.14 | 4.86 | 9.94 |
| $C_9$ Aromatics | 2.43 | 2.26 | 4.53 |
| $C_{10}$+ Aromatics | 0.64 | 0.66 | 1.38 |
| $C_1$—$C_5$ Paraffins | 13.28 | 37.21 | 24.27 |
| $C_2$—$C_5$ Olefins | 76.73 | 47.41 | 49.53 |
| $C_6$+ PON | 2.62 | 3.95 | 3.55 |
| Aromatics | 7.37 | 11.43 | 22.63 |
| % Conversion | 95.90 | 99.19 | 99.73 |

Example 4

The catalyst was prepared as in Example 1 except that $B_2O_3$ and liquid ammonia were added simultaneously as a saturated solution (0.2463 g in 3 g HZSM—5 or 4.5 B/Al) (α = 37). Table 2 gives details of the methanol conversion activity of the catalyst.

TABLE 2

| Example No. | 4 | 4 |
|---|---|---|
| Temperature °C | 370 | 500 |
| Pressure, atms. | 14.70 | 14.70 |
| (kPa) | 1485 | 1485 |
| LHSV | 1.00 | 1.00 |
| Time on Stream (TOS) | 5.00 | 2.55 |
| | Product Distribution | |
| $H_2O$ | 54.94 | 54.30 |
| DME | 0.65 | 0.38 |
| MeOH | 1.18 | 1.26 |
| CO | 0.08 | 0.20 |
| $CO_2$ | 0.02 | 0.08 |
| $H_2$ | 0.02 | 0.11 |
| HC | 49.11 | 49.67 |

TABLE 2 Continued

| Example No. | 4 | 4 |
|---|---|---|
| | Hydrocarbon Distribution | |
| Methane | 0.73 | 2.16 |
| Ethane | 0.17 | 0.77 |
| Ethylene | 10.31 | 16.49 |
| Propane | 6.02 | 8.86 |
| i-butane | 16.63 | 7.08 |
| n-butane | 2.18 | 2.46 |
| Butenes | 11.94 | 14.60 |
| i-pentane | 8.83 | 2.78 |
| n-pentane | 0.57 | 0.57 |
| Pentenes | 4.33 | 3.50 |
| $C_6$+ PON | 10.99 | 2.99 |
| Benzene | 0.35 | 0.34 |
| Toluene | 1.38 | 2.36 |
| $C_8$ Aromatics | 5.27 | 4.90 |
| $C_9$ Aromatics | 4.52 | 1.95 |
| $C_{10}$+ Aromatics | 1.83 | 0.64 |
| $C_1$—$C_5$ Paraffins | 35.12 | 24.67 |
| $C_2$—$C_5$ Olefins | 40.54 | 62.14 |
| $C_6$+ PON | 10.99 | 2.99 |
| Aromatics | 19.95 | 10.19 |
| % Conversion | 97.91 | 98.21 |

Example 5

The catalyst was prepared as in Example 1 except that 3 g HZSM—5 and 0.45 g $Ca(NO_3)_2 \cdot 4H_2O$ were used (Ca/Al=1.6) ($\alpha = 11$). The methanol conversion activity of the catalyst is illustrated by the results given in Table 3.

TABLE 3

| Example No. | 5 | 5 |
|---|---|---|
| Temperature °C | 450 | 500 |
| Pressure, atms. | 14.70 | 14.70 |
| (kPa) | 1485 | 1485 |
| LHSV | 1.00 | 1.00 |
| Time on Stream (TOS) | 3.75 | 5.75 |
| | Product Distribution | |
| $H_2$ | 50.87 | 56.56 |
| DME | 0.26 | 0.36 |
| MeOH | 1.75 | 0.11 |
| CO | 0.65 | 0.55 |
| $CO_2$ | 0.16 | 0.51 |
| $H_2$ | 0.11 | 0.15 |
| HC | 46.21 | 41.76 |
| | Hydrocarbon Distribution | |
| Methane | 1.42 | 1.81 |
| Ethane | 0.17 | 0.71 |
| Ethylene | 9.91 | 15.40 |
| Propane | 3.26 | 5.71 |
| Propylene | 29.21 | 28.39 |
| i-butane | 6.25 | 4.25 |
| n-butane | 1.28 | 1.50 |
| Butene | 20.54 | 16.55 |
| i-pentane | 5.46 | 2.25 |
| n-pentane | 0.61 | 0.47 |
| Pentenes | 6.11 | 4.90 |
| $C_6$+ PON | 8.48 | 6.31 |
| Benzene | 0.13 | 0.34 |
| Toluene | 1.05 | 2.72 |

TABLE 3 Continued

| Example No. | 5 | 5 |
|---|---|---|
| | Hydrocarbon Distribution | |
| $C_8$ Aromatics | 3.22 | 6.12 |
| $C_9$ Aromatics | 2.15 | 2.20 |
| $C_{10}$+ Aromatics | 0.73 | 0.37 |
| $C_1$—$C_5$ Paraffins | 18.44 | 16.70 |
| $C_2$—$C_5$ Olefins | 65.78 | 65.24 |
| $C_6$+ PON | 8.48 | 6.31 |
| Aromatics | 7.29 | 11.75 |
| % Conversion | 97.89 | 99.39 |

Example 6

Catalyst preparation as in Example 2 except only 3 g HZSM—5 were used (2:1 Mg:Al) (α = 53). The results of using the catalyst to effect conversion of methanol are shown in Table 4.

TABLE 4

| Example No. | 6 | 6 |
|---|---|---|
| Temperature °C | 370 | 500 |
| Pressure, atms. | 14.70 | 14.70 |
| (kPa) | 1485 | 1485 |
| LHSV | 1.00 | 1.00 |
| Time on Stream (TOS) | 3.00 | 5.75 |
| | Product Distribution | |
| $H_2$ | 50.28 | 56.32 |
| DME | 0.20 | 0.57 |
| MeOH | 2.94 | 0.27 |
| CO | 0.09 | 0.56 |
| $CO_2$ | 0.06 | 0.37 |
| $H_2$ | 0.01 | 0.30 |
| HC | 46.42 | 41.60 |
| | Hydrocarbon Distribution | |
| Methane | 0.63 | 2.16 |
| Ethane | 0.14 | 1.39 |
| Ethylene | 10.32 | 15.26 |
| Propane | 3.60 | 8.63 |

TABLE 4 Continued

| Example No. | 6 | 6 |
|---|---|---|
| | Hydrocarbon Distribution | |
| Propene | 10.02 | 21.28 |
| i-butane | 13.00 | 4.67 |
| n-butane | 1.98 | 2.23 |
| Butenes | 11.40 | 10.64 |
| i-pentane | 10.60 | 1.74 |
| n-pentane | 0.73 | 0.50 |
| Pentene | 4.39 | 2.40 |
| $C_6$+ PON | 18.15 | 4.83 |
| Benzene | 0.27 | 1.49 |
| Toluene | 0.89 | 6.81 |
| $C_8$ Aromatics | 5.44 | 11.09 |
| $C_9$ Aromatics | 6.08 | 4.25 |
| $C_{10}$+ Aromatics | 2.36 | 0.65 |
| $C_1$—$C_5$ Paraffins | 30.68 | 21.32 |
| $C_2$—$C_5$ Olefins | 36.18 | 49.58 |
| $C_6$+ PON | 18.15 | 4.83 |
| Aromatics | 15.04 | 24.28 |
| % Conversion | 96.78 | 98.94 |

**Claims**

1. A process for converting a charge comprising an alcohol and/or ether to an olefinic product in which the charge is contacted under conversion conditions with a catalyst comprising a crystalline aluminosilicate zeolite having incorporated therein at least one metal or metal compound by contacting the zeolite with a solution of the metal or metal compound and recovering the resultant metal-exchanged or loaded crystalline aluminosilicate zeolite, characterized in that the metal or metal compound is in solution in a non-aqueous inorganic solvent.

2. A process according to claim 1, wherein the non-aqueous inorganic solvent is liquid ammonia.

3. A process according to claim 1 or claim 2, wherein the zeolite is hydrophobic and has a silica-to-alumina ratio of at least 20.

4. A process according to any one of claims 1 to 3, wherein the metal is selected from Groups I and II of the Periodic Table.

5. A process according to claim 4, wherein the metal is selected from sodium, potassium, calcium, magnesium and barium.

**Patentansprüche**

1. Verfahren zur Umwandlung einer Charge, die einen Alkohol und/oder Äther umfaßt, zu einem olefinischen Produkt, worin die Charge unter Umwandlungsbedingungen mit einem Katalysator in Kontakt gebracht wird, der einen kristallinen Aluminosilikatzeolith umfaßt, der durch Kontakt des Zeolith mit einer Lösung des Metalls oder der Metallverbindung und durch Rückgewinnung des resultierenden mit Metall ausgetauschten oder beladenen kristallinen Aluminosilikatzeolith zumindest ein Metall oder eine

Metallverbindung darin eingearbeitet aufweist, dadurch gekennzeichnet, daß das Metall oder die Metallverbindung in Lösung in einem nichtwässrigen anorganischen Lösungsmittel verliegt.

2. Verfahren nach Anspruch 1, worin das nichtwässrige anorganische Lösungsmittel flüssiger Ammoniak ist.

3. Verfahren nach Anspruch 1 oder 2, worin der Zeolith hydrophob ist und ein Siliciumdioxid/Aluminiumoxid-Verhältnis von mindestens 20 hat.

4. Verfahren nach einem der Ansprüche 1 bis 3, worin das Metall aus den Gruppen I und II des Periodensystems ausgewählt ist.

5. Verfahren nach Anspruch 4, worin das Metall aus Natrium, Kalium, Kalzium, Magnesium und Barium ausgewählt ist.

**Revendications**

1. Procédé de transformation d'une charge comprenant un alcool et/ou un éther en un produit oléfinique dans lequel on met la charge dans des conditions de conversion au contact d'un catalyseur qui comprend une zéolite à base d'aluminosilicate cristallin dans laquelle sont incorporés, au moins, un métal ou un composé métallique par mise de la zéolite au contact d'une solution de métal ou de composé métallique et récupération de la zéolite d'aluminosilicate cristallin échangée par un métal ou chargée par un métal, caractérisé en ce que le métal ou le composé métallique se trouve en solution dans un solvant minéral non aqueux.

2. Procédé selon la revendication 1, dans lequel le solvant minéral non aqueux est l'ammoniac liquide.

3. Procédé selon la revendication 1 ou 2, dans lequel la zéolite est hydrophobe et son rapport silice/alumine est au moins égal à 20.

4. Procédé selon l'une quelconque des revendications 1 à 3, dans lequel le métal est choisi dans les groupes I et II de la Classification Périodique des Eléments.

5. Procédé selon la revendication 4, dans lequel le métal est choisi parmi le sodium, potassium, calcium, magnésium or baryum.